(19) Europäisches Patentamt
European Patent Office
Office européen des brevets

(11) **EP 1 525 172 B1**

(12) **EUROPÄISCHE PATENTSCHRIFT**

(45) Veröffentlichungstag und Bekanntmachung des Hinweises auf die Patenterteilung:
**02.11.2016 Patentblatt 2016/44**

(21) Anmeldenummer: **03763753.5**

(22) Anmeldetag: **09.07.2003**

(51) Int Cl.:
*C07C 45/33* [(2006.01)]  *C07C 45/35* [(2006.01)]
*C07C 51/215* [(2006.01)]  *C07C 51/25* [(2006.01)]
*C07C 45/34* [(2006.01)]

(86) Internationale Anmeldenummer:
**PCT/EP2003/007356**

(87) Internationale Veröffentlichungsnummer:
**WO 2004/007405 (22.01.2004 Gazette 2004/04)**

(54) **VERFAHREN ZUM SICHEREN BETREIBEN EINER KONTINUIERLICHEN HETEROGEN KATALYSIERTEN GASPHASEN-PARTIALOXIDATION WENIGSTENS EINER ORGANISCHEN VERBINDUNG**

METHOD FOR RELIABLY OPERATING A CONTINUOUS HETEROGENEOUSLY CATALYZED GAS PHASE PARTIAL OXIDATION OF AT LEAST ONE ORGANIC COMPOUND

PROCEDE D'EXPLOITATION FIABLE D'UNE OXYDATION PARTIELLE CONTINUE EN PHASE GAZEUSE D'AU MOINS UN COMPOSE ORGANIQUE, CATALYSEE DE MANIERE HETEROGENE,

(84) Benannte Vertragsstaaten:
**AT BE BG CH CY CZ DE DK EE ES FI FR GB GR HU IE IT LI LU MC NL PT RO SE SI SK TR**

(30) Priorität: **17.07.2002 DE 10232482**

(43) Veröffentlichungstag der Anmeldung:
**27.04.2005 Patentblatt 2005/17**

(73) Patentinhaber: **BASF SE**
**67056 Ludwigshafen am Rhein (DE)**

(72) Erfinder:
• **HAMMON, Ulrich**
**68163 Mannheim (DE)**
• **MESCHKE, Jochen**
**67166 Otterstadt (DE)**
• **RAUH, Ulrich**
**67227 Frankenthal (DE)**
• **MÜLLER-ENGEL, Klaus, Joachim**
**76297 Stutensee (DE)**
• **SCHLEMMER, Peter**
**67304 Eisenberg (DE)**
• **SCHLIEPHAKE, Volker**
**67105 Schifferstadt (DE)**

(56) Entgegenhaltungen:
**EP-A- 1 180 508    DE-A- 19 902 562**

**Beschreibung**

Die vorliegende Anmeldung betrifft ein Verfahren zum sicheren Betreiben einer kontinuierlich betriebenen heterogen katalysierten Gasphasen-Partialoxidation wenigstens einer organischen Verbindung in einem Oxidationsreaktor, dessen Beschickungsgasgemisch neben der wenigstens einen partiell zu oxidierenden organischen Verbindung und molekularem Sauerstoff als Oxidationsmittel wenigstens ein sich unter den Bedingungen der heterogen katalysierten Gasphasen-Partialoxidation im wesentlichen inert verhaltendes Verdünnungsgas umfasst, bei dem mittels eines Abschaltmechanismus verhindert wird, dass der Oxidationsreaktor mit einem Beschickungsgasgemisch beschickt wird, dessen Zusammensetzung eine explosive ist, dadurch gekennzeichnet , dass der Abschaltmechanismus wie folgt gestaltet ist:a) in einem Rechner wird ein für das Beschickungsgasgemisch charakteristisches Explosionsdiagramm hinterlegt, in welchem in Abhängigkeit von der Zusammensetzung des Beschickungsgasgemisches explosive und nicht explosive Zusammensetzungen gegeneinander abgrenzt sind;b) durch Bestimmungen bezüglich Menge und gegebenenfalls Zusammensetzung der dem Reaktor zur Erzeugung des Beschickungsgasgemisches zugeführten Gasströme wird ein Datensatz ermittelt, der an den Rechner weitergeleitet wird;c) aus dem unter b) erhaltenen Datensatz errechnet der Rechner einen aktuellen Betriebspunkt des Beschickungsgasgemisches im Explosionsdiagramm;d) fällt der Abstand des Betriebspunktes zur nächstliegenden Explosionsgrenze unter einen vorgegebenen Mindestwert, wird die Zufuhr von Gasströmen zum Reaktor automatisch unterbrochen,wobei der Sauerstoffgehalt des Beschickungsgasgemisches oberhalb der Sauerstoffgrenzkonzentration des Beschickungsgasgemisches liegt.

Unter einer vollständigen Oxidation einer organischen Verbindung mit molekularem Sauerstoff wird hier verstanden, daß die organische Verbindung unter reaktiver Einwirkung von molekularem Sauerstoff so umgesetzt wird, daß der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird. Alle davon verschiedenen Umsetzungen einer organischen Verbindung unter reaktiver Einwirkung von molekularem Sauerstoff werden hier als Partialoxidationen einer organischen Verbindung zusammengefaßt. Im besonderen sollen hier unter Partialoxidationen solche Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält als vor Durchführung der Partialoxidation.

Als ein sich unter den Bedingungen der heterogen katalysierten Gasphasen-Partialoxidation im wesentlichen inert verhaltendes Verdünnungsgas werden solche Verdünnungsgase verstanden, deren Bestandteile unter den Bedingungen der heterogen katalysierten Gasphasen-Partialoxidation - jeder Bestandteil für sich betrachtet - zu mehr als 95 mol-%, vorzugsweise zu mehr als 99 mol-% unverändert erhalten bleiben.

Gasförmige, molekularen Sauerstoff und eine partiell oxidierbare gasförmige organische Verbindung enthaltende, Gemische sind potentiell explosive Gasgemische.

Entscheidend für die Beantwortung der Frage, ob das Gasgemisch explosiv ist oder nicht, ist, ob sich in dem unter bestimmten Ausgangsbedingungen (Druck, Temperatur) befindlichen Gasgemisch eine durch eine örtliche Zündquelle (z.B. glühender Platindraht) eingeleitete Verbrennung (Entzündung, Explosion) ausbreitet oder nicht (vgl. DIN 51649). Erfolgt eine Ausbreitung, soll das Gemisch hier als explosiv bezeichnet werden. Erfolgt keine Ausbreitung, wird das Gemisch in dieser Schrift als nicht explosiv eingeordnet.

Die dazu erforderlichen Untersuchungen werden üblicherweise in einem geschlossenen 5l-Hochdruckbehälter aus Edelstahl durchgeführt. Die Erzeugung des Gasgemisches in dem anfangs evakuierten Hochdruckbehälter erfolgt üblicherweise nach der Partialdruckmethode. Nach einer Mischzeit von ca. 10 min. mittels eines Magnetrührwerkes wird versucht, das jeweils bei einem bestimmten Ausgangsdruck und einer bestimmten Ausgangstemperatur befindliche Gasgemisch mittels eines durchschmelzenden Platindrahtes zu zünden. Eine dadurch eventuell ausgelöste selbstständige Ausbreitung einer Reaktionsfront (Explosion) wird über den zeitlichen Anstieg des Behälterinnendruckes (z.B. Messung mit piezoelektrischem Druckaufnehmer) und über die Erhöhung der Temperatur im Behälter detektiert (vgl. z.B. EP-A 731080 und DE-A 19622331.

Gase, deren Gemische mit Luft bei jedem Mischungsverhältnis zu keiner sich von einer Zündquelle ausbreitenden Verbrennungsreaktion befähigt sind, sollen hier als nicht brennbare Gase bezeichnet werden. Typische Beispiele für nicht brennbare Gase sind $CO_2$, $H_2O$, $N_2$ und alle Edelgase.

Gase, deren Gemische mit Luft bei bestimmten Mischungsverhältnissen zu sich von einer Zündquelle ausbreitenden Verbrennungsreaktion befähigt sind, sollen hier als brennbare Gase bezeichnet werden. Beispiele für brennbare Gase sind Wasserstoff, Ethan, Propan, Butan oder Diethylether.

Während nicht brennbare Gase grundsätzlich für heterogen katalysierte Gasphasen-Partialoxidationen organischer Verbindungen geeignete inerte Verdünnungsgase sind, muß dies der Fachmann bei brennbaren Gasen bezüglich einer bestimmten heterogen katalysierten Gasphasen-Partialoxidation mittel weniger Vorversuche ermitteln.

Es ist allgemein bekannt, daß durch partielle und heterogen katalysierte Oxidation verschiedener organischer Verbindungen mit molekularem Sauerstoff in der Gasphase zahlreiche Grundchemikalien erzeugt werden können. Beispielhaft genannt seien die Umsetzung von Propylen zu Acrolein und/oder Acrylsäure (vgl. z.B.

DE-A 2351151), die Umsetzung von tert. Butanol, iso-Buten, iso-Butan, iso-Butyraldehyd oder dem Methylether des tert. Butanols zu Methacrolein und/oder Methacrylsäure (vgl. z.B. DE-A 2526238, EP-A 92097, EP-A 58927, DE-A 4132263, DE-A 4132684 und DE-A 4022212), die Umsetzung von Acrolein zu Acrylsäure, die Umsetzung von Methacrolein zu Methacrylsäure (vgl. z.B.

DE-A 2526238), die Umsetzung von o-Xylol oder Naphthalin zu Phthalsäureanhydrid (vgl. z.B. EP-A 522871) sowie die Umsetzung von Butadien zu Maleinsäureanhydrid (vgl. z.B. DE-A 2106796 und DE-A 1624921), die Umsetzung von n-Butan zu Maleinsäureanhydrid (vgl. z.B. GB-A 1464198 und GB-A 1291354), die Umsetzung von Indanen zu z.B. Anthrachinon (vgl. z.B. DE-A 2025430), die Umsetzung von Ethylen zu Ethylenoxid oder von Propylen zu Propylenoxid (vgl. z.B. DE-AS 1254137, DE-A 2159346, EP-A 372972, WO 89/0710, DE-A 4311608 und Beyer, Lehrbuch der organischen Chemie, 17. Auflage (1973), Hirzel Verlag Stuttgart, S. 261), die Umsetzung von Propylen und/oder Acrolein zu Acrylnitril (vgl. z.B.

DE-A 2351151), die Umsetzung von iso-Buten und/oder Methacrolein zu Methacrylnitril (d.h., der Begriff der partiellen Oxidation soll in dieser Schrift auch die partielle Ammoxidation, d.h., eine partielle Oxidation im Beisein von Ammoniak, umfassen), die oxidative Dehydrierung von Kohlenwasserstoffen (vgl. z.B. DE-A 2351151), die Umsetzung von Propan zu Acrylnitril oder zu Acrolein und/oder Acrylsäure (vgl. z.B. DE-A 10131297, EP-A 1090684, EP-A 608838, DE-A 10046672, EP-A 529853, WO 01/96270 und DE-A 10028582) etc..

Bei den zu verwendenden Katalysatoren handelt es sich normalerweise um Festkörper.

Besonders häufig handelt es sich bei den verwendeten Katalysatoren um Oxidmassen oder um Edelmetalle (z.B. Ag). Die katalytisch aktive Oxidmasse kann neben Sauerstoff lediglich ein anderes Element oder mehr als ein anderes Element (Multielementoxidmassen) enthalten. Besonders häufig kommen als katalytisch aktive Oxidmassen solche zur Anwendung, die mehr als ein metallisches, insbesondere übergangsmetallisches, Element umfassen. In diesem Fall spricht man von Multimetalloxidmassen. Üblicherweise sind Multielementoxidmassen keine einfachen physikalischen Gemische von Oxiden der elementaren Konstituenten, sondern heterogene Gemische von komplexen Polyverbindungen dieser Elemente.

Meist werden heterogen katalysierte Gasphasen-Partialoxidationen, insbesondere die vorgenannten, bei erhöhter Temperatur (in der Regel einige hundert °C, überlicherweise 100 bis 600°C) durchgeführt.

Da die meisten heterogen katalysierten Gasphasen-Partialoxidationen stark exotherm verlaufen, führt man sie aus Gründen der Wärmeabfuhr zweckmäßigerweise häufig im Wirbelbett oder in Vielkontaktrohr-Festbettreaktoren durch, durch deren die Kontaktrohre umgebenden Raum ein Wärmeaustauschmittel geleitet wird.

Der Arbeitsdruck (Absolutdruck) bei heterogen katalysierten Gasphasen-Partialoxidationen kann sowohl unter 1 atm, bei 1 atm oder über 1 atm liegen. In der Regel beträgt er 1 bis 10 atm, meist 1 bis 3 atm. Die Zielumsetzung erfolgt während der Verweilzeit des Reaktionsgasgemisches in der Katalysatorbeschickung durch die es geleitet wird.

Aufgrund des in der Regel ausgeprägt exothermen Charakters der meisten heterogen katalysierten Gasphasen-Partialoxidationen organischer Verbindungen mit molekularem Sauerstoff werden die Reaktionspartner üblicherweise mit einem sich unter den Bedingungen der gasphasenkatalytischen Partialoxidation im wesentlichen inerten Gas verdünnt, das mit seiner Wärmekapazität frei werdende Reaktionswärme zu absorbieren vermag.

Eines der häufigsten mitverwendeten inerten Verdünnungsgase ist molekularer Stickstoff, der automatisch immer dann zur Anwendung kommt, wenn als Sauerstoffquelle für die heterogen katalysierte Gasphasen-Partialoxidation Luft verwendet wird.

Ein anderes vielfach mitverwendetes inertes Verdünnungsgas ist wegen seiner allgemeinen Verfügbarkeit Wasserdampf. Sowohl Stickstoff als auch Wasserdampf bilden zudem in vorteilhafter Weise nicht brennbare inerte Verdünnungsgase.

Vielfach wird auch Kreisgas als inertes Verdünnungsgas mitverwendet (vgl. z.B. EP-A 1180508). Als Kreisgas wird das Restgas bezeichnet, das nach einer einstufigen oder mehrstufigen (bei der mehrstufigen heterogen katalysierten Gasphasen-Partialoxidation organischer Verbindungen wird die Gasphasen-Partialoxidation im Unterschied zur einstufigen heterogen katalysierten Gasphasen-Partialoxidation nicht in einem, sondern in wenigstens zwei hintereinandergeschalteten Reaktoren durchgeführt, wobei zwischen aufeinanderfolgenden Reaktoren in der Regel Oxidationsmittel ergänzt wird; die Mehrstufigkeit wird insbesondere dann angewandt, wenn sich die Partialoxidation in aufeinanderfolgenden Schritten vollzieht; in diesen Fällen ist es häufig zweckmäßig, sowohl den Katalysator als auch die sonstigen Reaktionsbedingungen an den jeweiligen Reaktionsschritt optimierend anzupassen, und den Reaktionsschritt in einem eigenen Reaktor, in einer separaten Reaktionsstufe durchzuführen; sie kann aber auch dann angewandt werden, wenn aus Gründen der Wärmeabfuhr oder aus anderen Gründen (vgl. z.B. DE-A 19902562) der Umsatz auf mehrere hinterandergeschaltete Reaktoren verschmiert wird; ein Beispiel für eine häufig zweistufig durchgeführte heterogen katalysierte Gasphasen-Partialoxidation ist die Partialoxidation von Propylen zu Acrylsäure; in der ersten Reaktionsstufe wird das Propylen zum Acrolein, und in der zweiten Reaktionsstufe das Acrolein zur Acrylsäure oxidiert; in entsprechender Weise wird auch häufig die Methacrylsäureherstellung, meist ausgehend von iso-Buten, zweistufig durchgeführt; beide vorgenannten Partialoxidationen können bei Verwendung geeigneter Katalysatorbeschickungen aber auch einstufig (beide Schritte in einem Reaktor) durchgeführt werden) heterogen katalysierten Gasphasen-Partialoxidation wenigstens einer organischen Verbindung dann verbleibt, wenn man aus dem Produktgasgemisch das Zielprodukt mehr oder weniger selektiv (z.B. durch Absorption in ein geeignetes Lösungsmittel) abgetrennt hat. Im Regelfall besteht es überwiegend aus den für die Partialoxidation verwendeten inerten Verdünnungsgasen sowie aus bei der Partialoxidation üblicherweise als Nebenprodukt gebildetem Wasserdampf und durch unerwünschte vollständige Oxidation gebildeten Kohlenoxiden und Wasserdampf. Teilweise enthält es noch geringe Mengen an bei der Partialoxidation nicht verbrauchtem Sauerstoff (Restsauerstoff) und/oder an nicht umgesetzten organischen Ausgangsverbindungen.

Der als Nebenprodukt gebildete Wasserdampf gewährleistet in den meisten Fällen, daß die Partialoxidation ohne signifikante Volumenänderungen des Reaktionsgasgemisches verläuft.

Gemäß dem Vorstehenden besteht somit bei den meisten heterogen katalysierten Gasphasen-Partialoxidationen organischer Verbindungen das mitverwendete inerte Verdünnungsgas zu $\geq$ 90 Vol.-%, häufig zu $\geq$ 95 Vol.-%, aus $N_2$, $H_2O$, und/oder $CO_2$ und damit im wesentlichen aus nicht brennbaren inerten Verdünnungsgasen.

Die mitverwendeten inerten Verdünnungsgase gewährleisten aber nicht nur im Verbund mit den sonstigen zur Abfuhr der Reaktionswärme angewandten Maßnahmen (z.B. externe Kühlung) eine weitgehend einheitliche Temperatur des Reaktionsgasgemisches längs des Reaktionspfades, sondern sie ermöglichen auch den sicheren Betrieb einer kontinuierlichen heterogen katalysierten Gasphasen-Partialoxidation von organischen Verbindungen.

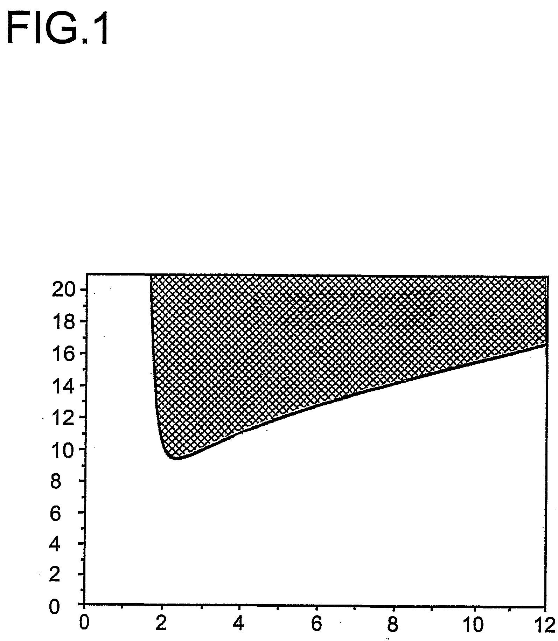

Untersucht man nämlich das Explosionsverhalten eines aus molekularem Sauerstoff, einem nichtbrennbaren inerten Verdünnungsgas und einer brennbaren organischen Verbindung bestehenden, bei einer bestimmten Temperatur und einem bestimmten Druck befindlichen, Gasgemisch in Abhängigkeit von der Gemischzusammensetzung, so erhält man ein Ergebnis, wie es in tpyischer Weise das Explosionsdiagramm der Figur 1 für das Gasgemisch Propylen/Sauerstoff-Stickstoff zeigt.

Die Abszisse zeigt den Anteil des Propylens am Gemisch in Vol.-% ($V_{C3}$), die Ordinate zeigt den Anteil des molekularen Sauerstoff am Gemisch in Vol.-% ($V_{O2}$) und die Restmenge bis 100 Vol.-% ist stets der molekulare Stickstoff.

Liegt die Zusammensetzung des Gasgemisches im schraffierten Bereich der Figur 1, ist es explosiv, liegt sie außerhalb, ist es nicht explosiv. Die durchgezogene Linie trennt den explosiven Bereich vom nichtexplosiven Bereich und wird als Explosionsgrenze bezeichnet.

Würde anstelle von Stickstoff z.B. Wasserdampf oder ein Gemisch aus Stickstoff und Wasserdampf als inertes Verdünnungsgas verwendet, würde die Explosionsgrenze nahezu deckungsgleich verlaufen. Dies gilt so auch für die meisten anderen nicht brennbaren inerten Verdünnungsgase, so lange ihre spezifische molare Wärme nicht erheblich von jener des molekularen Stickstoff abweicht. Die Druck- und Temperaturabhängigkeit von Explosionsdiagrammen ist nur begrenzt ausgeprägt und bei nicht zu großen Änderungen vernachläßigbar.

Würde das Propylen durch ein Gemisch von organischen Verbindungen bestimmter Zusammensetzung oder durch eine andere organische Verbindung ersetzt, wäre der qualitative Verlauf der Explosionsgrenze im Explosionsdiagramm qualitativ der gleiche.

Von Vorteil für die vorliegende Erfindung ist, daß wenn das Reaktionsgasausgangsgemisch (das Beschickungsgasgemisch) außerhalb des explosiven Bereichs liegt, normalerweise auch alle Reaktionsgasgemische längs des Reaktionspfades der heterogen katalysierten Gasphasenpartialoxidation außerhalb des explosiven Bereichs liegen. Dies ist einerseits darauf zurückzuführen, daß Druck und Temperatur längs des Reaktionspfades nur vergleichsweise begrenzt variieren und andererseits das partielle Oxidationsprodukt (hier z.B. Acrolein) infolge seiner Ähnlichkeit zur Ausgangsverbindung (hier: Propylen) ein im wesentlichen vergleichbares Explosionsdiagramm wie die Ausgangsverbindung selbst zeigt. Zusätzlich nimmt der prozentuale Sauerstoffgehalt längs des Reaktionspfades auf dem Weg von der Ausgangsverbindung zum Zielprodukt ab. Zum einen aufgrund seines Verbrauchs und zum anderen häufig durch zusätzliche Verdünnung infolge des als Nebenprodukt gebildeten Oxidationswassers. Außerdem handelt es sich bei Verbrennungen (Explosionen) um radikalische Kettenreaktionen. Die verwendeten Katalysatoren wirken mit ihrer großen Oberfläche diesbezüglich als Radikalfänger.

D.h., bei den meisten kontinuierlich betriebenen heterogen katalysierten Gasphasen-Partialoxidationen wenigstens einer organischen Verbindung in einem Oxidationsreaktor ist ein Betrieb mit erhöhter Sicherheit dann gewährleistet, wenn die Zusammensetzung des Reaktionsgasausgangsgemisches während des kontinuierlichen Betriebs (insbesondere im stationären Zustand) bei einem für die Partialoxidation repräsentativen Druck und Temperatur stets außerhalb des Explosionsbereichs liegt.

Zwar ist prinzipiell auch ein Betrieb mit einem Reaktionsgasausgangsgemisch möglich, dessen Zusammensetzung innerhalb des Explosionsbereichs liegt. Allerdings kann in diesem Fall jedwede lokale Zündquelle (z.B. lokale Überhitzung oder Funkenbildung im Reaktor) zur unerwünschten Explosion führen, der durch aufwendige Sicherheitseinrichtungen (z.B. Berstscheiben, Sicherheitsventile, Flammenrückschlagssicherungen) und druckfeste Reaktoren begegnet werden müßte, die vergleichsweise teuer und auch aufwendig in der Wartung sind. Im Fall von toxischen Hauptund Nebenprodukten wären jedoch selbst vorgenannte Begegnungsmaßnahmen nicht ausreichend.

Wesentlich für einen kontinuierlichen Betrieb einer heterogen katalysierten Gasphasen-Partialoxidation wenigstens einer organischen Verbindung in einem Oxidationsreaktor mit außerhalb des Explosionsbereichs liegenden Zusammensetzungen des Reaktionsgasausgangsgemisches ist jedoch, daß der kontinuierliche Betrieb über einen Abschaltmechanismus verfügt, der verhindert, daß die außerhalb des Explosionsbereichs liegende Zusammensetzung des Reaktionsgasausgangsgemisches (Beschickungsgasgemisches) während des kontinuierlichen Betriebs (d.h., insbesondere im stationären Betrieb) durch unbeabsichtigte Störungen in den dem Reaktor zur Bildung des Beschickungsgasgemisches zugeführten Gasströmen (diese bestehen in der Regel aus Luft, der zu oxidierenden Substanz sowie inertem Verdünnungsgas und/oder gegebenenfalls Kreisgas) in den Explosionsbereich hinein wandert.

Ein besonders einfacher Abschaltmechanismus besteht darin, den Gehalt des Beschickungsgasgemisches an der wenigstens einen partiell zu oxidierenden organischen Verbindung stets unterhalb deren Grenzkonzentration zu halten. Unterhalb der Grenzkonzentration liegt der Gehalt des Beschickungsgasgemisches an der wenigstens einen partiell zu oxidierenden organischen Verbindung dann, wenn bei einem vorgegebenen Gehalt des Beschickungsgasgemisches an der wenigstens einen partiell zu oxidierenden organischen Verbindung die Zusammensetzung des Beschickungsgasgemisches (bei vorgegebenem Druck und Temperatur) unabhängig von der Mengenzusammensetzung ihrer übrigen Bestandteile ($O_2$/inertes Verdünnungsgas) stets außerhalb des Explosionsbereichs liegt.

In Figur 1 beträgt die Propylengrenzkonzentration etwa 2 Vol.-% (vgl. auch EP-B 1180508, Seite 8, Zeile 32).

D.h., hält man bei der heterogen katalysierten Partialoxidation von Propylen zu Acrolein und/oder Acrylsäure unter Verwendung von $N_2$ und/oder Wasserdampf als Inertgas die Propylenkonzentration des Beschickungsgasgemisches unterhalb von 2 Vol.-%, ist das Beschickungsgasgemisch unabhängig davon, wie sich die verbleibenden > 98 Vol.-% des Beschickungsgasgemisches aus $O_2$, $N_2$ und $H_2O$ zusammensetzen, außerhalb des Explosionsbereichs.

Als Abschaltmechanismus ist es im vorgenannten Fall ausreichend, die Propylenkonzentration im Beschickungsgasgemisch über die Zeit zu verfolgen. Nähert sich diese der 2 Vol.-% Grenze, wird die Propylenzufuhr aus Sicherheitsgründen abgeschaltet.

Nachteilig an diesem Sicherheitskonzept ist, daß die damit erzielbare Raum-Zeit-Ausbeute infolge der Begrenzung der Propenkonzentration im Beschickungsgasgemisch selbst eine vergleichsweise begrenzte ist und nicht zu befriedigen vermag.

Als Verbesserung empfiehlt die EP-B 1180508 den stationären kontinuierlichen Betrieb einer heterogen katalysierten Gasphasen-Partialoxidation wenigstens einer organischen Verbindung in einem Oxidationsreaktor so durchzuführen (am Beispiel der Propenoxidation zu Acrolein und/oder Acrylsäure), daß der Gehalt des Beschickungsgasgemisches an molekularem Sauerstoff stets unterhalb der Sauerstoffgrenzkonzentration liegt (S. 8, Zeilen 28 bis 34 der EP-A 1180508). Diese Empfehlung gibt auch die EP-A 731080 auf Seite 3, Zeilen 29/30.

Unterhalb der Sauerstoffgrenzkonzentration liegt der $O_2$-Gehalt des Beschickungsgasgemisches dann, wenn bei einem vorgegebenen $O_2$-Gehalt des Beschickungsgasgemisches die Zusammensetzung des Beschickungsgasgemisches (bei vorgegebenem Druck und Temperatur) unabhängig von der Mengenzusammensetzung ihrer übrigen Bestandteile (wenigstens eine partiell zu oxidierende organische Verbindung/inertes Verdünnungsgas) stets außerhalb des Explosionsbereichs liegt.

In Figur 1 (sie bezieht sich auf 250°C und 1 atm) beträgt die Sauerstoffgrenzkonzentration etwa 10 Vol.-% (vgl. auch EP-B 1180508, Seite 8, Zeile 32).

D.h., hält man bei der heterogen katalysierten Partialoxidation von Propylen zu Acrolein und/oder Acrylsäure unter Verwendung von $N_2$ und/oder Wasserdampf als Inertgas die Sauerstoffkonzentration des Beschickungsgasgemisches unterhalb von 10 Vol.-%, ist das Beschickungsgasgemisch unabhängig davon, wie sich die verbleibenden > 90 Vol.-% des Beschickungsgasgemisches aus Propylen und $N_2$/$H_2O$ zusammensetzen, außerhalb des Explosionsbereichs.

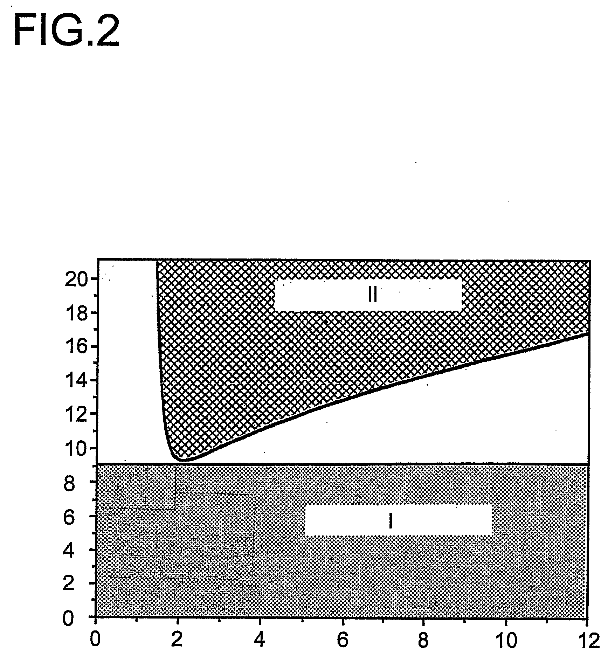

Damit steht als mit erhöhter Sicherheit betreibbarer Betriebsbereich der gesamte Bereich I in Figur 2 zur Verfügung. Der Bereich II in Figur 2 ist der explosive Bereich, die Abszisse ist der Propylengehalt in Vol.-% und die Ordinate ist der Sauerstoffgehalt in Vol.-%. Die Restmenge bis 100 Vol.-% ist inertes Verdünnungsgas aus $N_2$ und/oder $H_2O$.

Als Abschaltmechanismus ist es im vorgenannten Fall ausreichend, die Sauerstoffkonzentration im Beschickungsgasgemisch über die Zeit zu verfolgen. Nähert sich diese der 10 Vol.-% Grenze, wird die Sauerstoffzufuhr aus Sicherheitsgründen abgeschaltet.

Nachteilig an diesem Sicherheitskonzept ist, daß es einerseits bezüglich der Stöchiometrie der angestrebten Partialoxidation zur gewünschten Zielverbindung, aber auch aus anderen Gründen, in der Regel erforderlich ist, den als Oxidationsmittel verwendeten molekularen Sauerstoff in wenigstens stöchiometrischen oder in überstöchiometrischen Mengen einzusetzen (z.B. aus Gründen der Re-Oxidation der als Katalysator eingesetzten oxidischen Masse, sowie zur Minderung von Kohlenstoffabscheidungen am Katalysator).

Mit einer Begrenzung der Sauerstoffkonzentration im Beschickungsgasgemisch geht somit unabdingbar ebenfalls eine signifikante Begrenzung der Raum-Zeit-Ausbeute einher.

Die Aufgabe der vorliegenden Erfindung bestand daher darin, ein Verfahren zum sicheren betreiben einer kontinuierlich betriebenen heterogen katalysierten Gasphasen-Partialoxidation wenigstens einer organischen Verbindung (insbesondere aller eingangs zitierten) in einem Oxidationsreaktor, dessen Beschickungsgasgemisch neben der wenigstens einen partiell zu oxidierenden organischen Verbindung und molekularem Sauerstoff als Oxidationsmittel wenigstens ein sich unter den Bedingungen der heterogen katalysierten Gasphasen-Partialoxidation im wesentlichen inert verhaltendes Verdünnungsgas umfaßt, mit einem Abschaltmechanismus zur Verfügung zu stellen, der in einer gegenüber dem Stand der Technik verbesserten Weise verhindert, daß der Oxidationsreaktor mit einem Beschickungsgasgemisch beschickt wird, dessen Zusammensetzung eine explosive ist, und zum Beispiel einen Betrieb mit Beschickungsgasen zuläßt, deren Zusammensetzung in Figur 2 in der zwischen der Unterkante der Fläche II und der Oberkante der Fläche I gelegenen weißen Fläche liegt.

Diezbezüglich wurde u.a. auch über Abschaltmechnismen nachgedacht, die auf mehr als nur einer einzuhaltenden Grenzgröße basieren. So ist es z.B. prinzipiell möglich, bei einer heterogen katalysierten Gasphasenpartialoxidation von Propylen zu Acrolein und/oder Acrylsäure (mit einem Propylenumsatz von $\geq$ 95 mol-% und einer Selektivität der Zielproduktbildung von $\geq$ 90 mol-%), bei der als Rohstoff Propylen (Roh-Propylen), als Sauerstoffquelle Luft und als zusätzliche Inertgasquelle dem Oxidationsreaktor Kreisgas zur Erzeugung des Beschickungsgasgemisches zugeführt wird, für folgende Größen Abschaltgrenzen vorzusehen:

a) die pro Zeiteinheit dem Reaktor maximal zuzuführende Menge an Luft ($V_L)_i$;

b) das maximale Verhältnis aus pro Zeiteinheit dem Reaktor zugeführter Menge an Luft zu dem Reaktor pro Zeiteinheit zugeführter Menge an Propylen ($Z_{L/P}$) ;

c) die dem Reaktor pro Zeiteinheit minimal zuzuführende Kreisgasmenge ($V_{Kr}$); und

d) die maximale Konzentration des Restsauerstoffgehaltes im Kreisgas ($CO_2$).

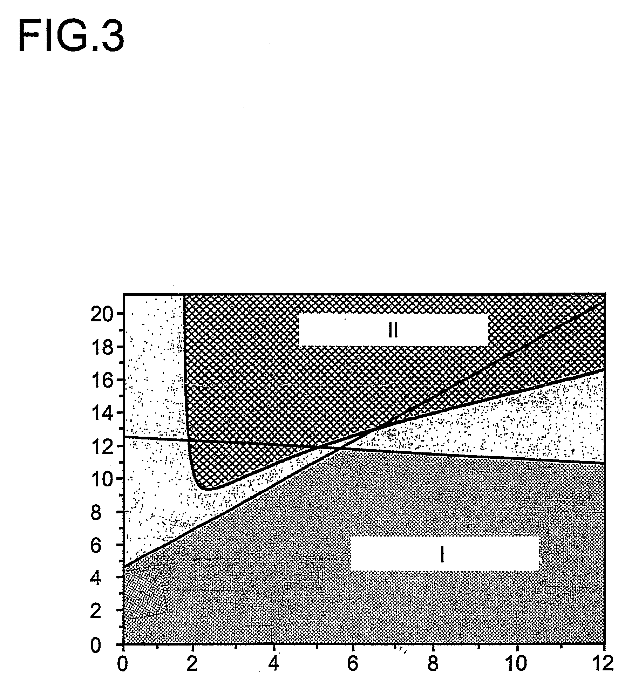

Wird einer der vier Grenzwerte angenähert (bezogen auf den Grenzwert sollte der Abstand stets möglichst $\geq$ 10 % sein), wird die Luftzufuhr aus Sicherheitsgründen abgeschaltet. In der Regel wird dazu zeitversetzt auch die Propylenzufuhr unterbrochen. Den dadurch mit erhöhter Betriebssicherheit zugänglichen Betriebsbereich zeigt die Figur 3 (die Bedeutung der Achsen und der numerischen Symbole ist wie in Figur 2).

Die flachere Gerade in Figur 3 genügt dabei der Gleichung:

$$V_{O2} = (1 - V_{C3}) \cdot \frac{A}{B} \, ,$$

mit $A = C_{O2} \cdot V_{Kr} + 0{,}21 \cdot V_L$
und $B = V_L + V_{Kr}$.

Die steile Gerade in Figur 3 genügt dabei der Gleichung:

$$V_{O2} = C_{O2} + V_{C3} \, (Z_{L/P} \cdot (0{,}21 - C_{O2}) - C_{O2}) .$$

Werden die Abschaltgrenzen nicht überschritten, kann das Beschikkungsgasgemisch keine der beiden Geraden zu höheren $V_{O2}$-Werten hin verlassen, so daß als Betriebsbereich der durch die "I" im Figur 3 charakterisierte schraffierte Bereich verbleibt. Er umfaßt zwar einen kleinen Bereich, bei dem der Sauerstoffgehalt im Beschickungsgasgemisch oberhalb der Sauerstoffgrenzkonzentration liegt, doch vermag dessen Ausprägung nicht im vollen Umfang zu befriedigen.

Deshalb wurde als Lösung der gestellten Aufgabe ein Verfahren zum Betreiben einer kontinuierlich betriebenen heterogen katalysierten Gasphasen-Partialoxidation wenigstens einer organischen Ver bindung mit erhöhter Sicherheit in einem Oxidationsreaktor, dessen Beschickungsgasgemisch neben der wenigstens einen partiell zu oxidierenden organischen Verbindung und molekularem Sauerstoff als Oxidationsmittel wenigstens ein sich unter den Bedingungen der heterogen katalysierten Gasphasen-Partialoxidation im wesentlichen inert verhaltendes Verdünnungsgas umfaßt und bei dem mittels eines Abschaltmechanismus verhindert wird, daß der Oxidationsreaktor mit einem Beschickungsgasgemisch beschickt wird, dessen Zusammensetzung eine explosive ist, gefunden, das dadurch gekennzeichnet ist, daß der Abschaltmechanismus wie folgt gestaltet ist:

a) in einem Rechner wird ein für das Beschickungsgasgemisch charakteristisches Explosionsdiagramm (z.B. die Figur 1) hinterlegt, in welchem in Abhängigkeit von der Zusammensetzung des Beschickungsgasgemisches explosive und nicht explosive Zusammensetzungen gegeneinander abgegrenzt sind;

b) durch Bestimmungen bezüglich Menge und gegebenenfalls Zusammensetzung der dem Reaktor zur Erzeugung des Beschikkungsgasgemisches zugeführten Gasströme wird ein Datensatz ermittelt, der an den Rechner weitergeleitet wird;

c) aus dem unter b) erhaltenen Datensatz errechnet der Rechner einen aktuellen Betriebspunkt des Beschickungsgasgemisches im Explosionsdiagramm;

d) fällt der Abstand des Betriebspunktes zur nächstliegenden Explosionsgrenze unter einen vorgegebenen Mindestwert, wird die Zufuhr von Gasströmen zum Reaktor automatisch unterbrochen.

Bevorzugt wird der Mindestwert aus einer statistischen Fehlerbetrachtung der zur Berechnung des Betriebspunktes notwendigen Meßgrößen berechnet.

Das erfindungsgemäße Verfahren gestattet es erstmals, heterogen katalysierte Gasphasen-Partialoxidationen wenigstens einer organischen Verbindung mit erhöhter Sicherheit bei Sauerstoffgehalten des Beschickungsgasgemisches durchzuführen, die $\geq 0,5$, oder $\geq 0,75$, oder $\geq 1$, oder $\geq 2$, oder $\geq 3$, oder $\geq 5$, oder $\geq 10$ Volumenprozentpunkte oberhalb der Sauerstoffgrenzkonzentration liegen. Unter der Sauerstoffgrenzkonzentration versteht man dabei, wie bereits beschrieben, denjenigen prozentualen Volumenanteil an molekularem Sauerstoff des Beschickungsgasgemisches, bei dessen Unterschreiten unabhängig von der Quantität der Volumenanteile der anderen Bestandteile des Beschickungsgasgemisches, nämlich insbesondere der partiell zu oxidierenden organischen Verbindung so wie dem inerten Verdünnungsgas, eine durch eine örtliche Zündquelle (wie z.B. lokale Überhitzung oder Funkenbildung im Reaktor) eingeleitete Verbrennung (Explosion) bei gegebenem Druck und Temperatur des Beschickungsgasgemisches (Bezugswerte sollen Temperatur und Druck des Beschickungsgasgemisches bei Eintritt in die Katalysatorbeschickung sein) sich in selbigem nicht mehr von der Zündquelle her auszubreiten vermag.

Aus Sicherheitsgründen kann es zweckmäßig sein, als Explosionsdiagramm nicht den Verlauf der experimentell ermittelten Explosionsgrenze sondern eine um einen Sicherheitsabstand zu dieser nach unten verschobene sogenannte Schaltkurve im Rechner zu hinterlegen. Der Sicherheitsabstand wird dabei in zweckmäßiger Weise so gewählt, dass alle bei der Ermittlung des Betriebspunktes des Beschickungsgasgemisches eingehenden Fehlerquellen und Meßungenauigkeiten damit berücksichtigt werden. Der Sicherheitsabstand kann dabei sowohl durch eine Absolutfehlerbetrachtung als auch durch eine statistische Fehlerbetrachtung ermittelt werden. In der Regel ist ein Sicherheitsabstand von 0,1 bis 0,4 Vol.-%-Punkten $O_2$ ausreichend.

Der Begriff Reaktor soll hier keine Laborreaktoren sondern nur Reaktoren im technischen Maßstab umfassen, da bei Laborreaktoren der zu praktizierende Sicherheitsrahmen ein anderer ist. Demnach sollen die hier betroffenen Reaktoren eine jährliche Produktionsleistung (bezogen auf 8000 Betriebsstunden pro Jahr) von wenigstens 2000 Tonnen Zielprodukt aufweisen.

D.h., im Fall von Kontaktrohrbündelreaktoren wird die Anzahl der Kontaktrohre von in dieser Schrift betroffenen Reaktoren in der Regel 500 bis 50000, häufig 10000 bis 30000 betragen.

Das erfindungsgemäße Verfahren eignet sich grundsätzlich für alle eingangs dieser Schrift spezifisch aufgeführten heterogen katalysierten Partialoxidationen.

Es eignet sich aber insbesondere für die im Rohrbündelreaktor einstufig durchgeführte heterogen katalysierte Partialoxidation von Propylen zu Acrolein und/oder Acrylsäure und für die erste Stufe einer in Rohrbündelreaktoren mehrstufig durchgeführten heterogen katalysierten Partialoxidation von Propylen zu Acrolein und/oder Acrylsäure, wie sie in den Schriften EP-A 700893, EP-A 700714, DE-A 19910508 und DE-A 19919596 beschrieben sind.

Als Rohstoff wird für dieses Verfahren in der Regel kein reines Propylen sondern sogenanntes Roh-Propen verwendet, das neben Propylen noch mehr oder weniger an anderen organischen Verbindungen enthält. Dieses Roh-Propen kann z.B. aus Naphtha-Crackern oder aus Dehydrierungen abgezweigtes Roh-Propen sein und beispielsweise die nachfolgenden Spezifikationen aufweisen:

a) Polymer grade Propylen:

| $\geq 99,6$ Gew.-% | Propen, |
|---|---|
| $\leq 0,4$ Gew-.% | Propan, |
| $\leq 300$ Gew.-ppm | Ethan und/oder Methan, |
| $\leq 5$ Gew.-ppm | $C_4$-Kohlenwasserstoffe, |
| $\leq 1$ Gew.ppm | Acetylen, |
| $\leq 7$ Gew.-ppm | Ethylen, |
| $\leq 5$ Gew.-ppm | Wasser, |
| $\leq 2$ Gew.-ppm | $O_2$, |

| | |
|---|---|
| ≤ 2 Gew.-ppm | Schwefel enthaltende Verbindungen (berechnet als Schwefel), |
| ≤ 1 Gew.-ppm | Chlor enthaltende Verbindungen (berechnet als Chlor), |
| ≤ 5 Gew.-ppm | $CO_2$, |
| ≤ 5 Gew.-ppm | CO, |
| ≤ 10 Gew.-ppm | Cyclopropan, |
| ≤ 5 Gew.-ppm | Propadien und/oder Propin, |
| ≤ 10 Gew.-ppm | C≥5-Kohlenwasserstoffe und |
| ≤ 10 Gew.-ppm | Carbonylgruppen enthaltende Verbindungen (berechnet als $Ni(CO)_4$); |

b) Chemical grade Propylen:

| | |
|---|---|
| ≥ 93 Gew.-% | Propen, |
| ≤ 7 Gew.-% | Propan, |
| ≤ 0,2 Gew.-% | Methan und/oder Ethan, |
| ≤ 5 Gew.-ppm | Ethylen, |
| ≤ 20 Gew.-ppm | Propadien und/oder Propin, |
| ≤ 100 Gew.-ppm | Cyclopropan, |
| ≤ 50 Gew.-ppm | Buten, |
| ≤ 50 Gew.-ppm | Butadien, |
| ≤ 200 Gew.-ppm | $C_4$-Kohlenwasserstoffe, |
| ≤ 10 Gew.-ppm | $C_{\geq 5}$-Kohlenwasserstoffe, |
| ≤ 2 Gew.-ppm | Schwefel enthaltende Verbindungen (berechnet als Schwefel), |
| ≤ 0,1 Gew.-ppm | Sulfide (berechnet als $H_2S$), |
| ≤ 1 Gew.-ppm | Chlor enthaltende Verbindungen (berechnet als Chlor), |
| ≤ 1 Gew.-ppm | Chloride (berechnet als $CL^{\theta}$) und |
| ≤ 30 Gew.-ppm | Wasser. |

Da sich Propan bezüglich der heterogen katalysierten Partialoxidation von Propylen in der Regel als Inertgas verhält, kann der Propangehalt im Roh-Propylen aber auch bei höheren Anteilen liegen.

Als Sauerstoffquelle ist es für das in Rede stehende Verfahren zweckmäßig, Luft (d.h., ein Gemisch, das im wesentlichen aus 21 Vol.-% molekularem Sauerstoff und aus 79 Vol.-% molekularem Stickstoff besteht) zu verwenden. Der den molekularen Sauerstoff in Luft begleitende molekulare Stickstoff bildet automatisch Verdünnungsgas. Als weiteres Verdünnungsgas wird in der Regel Wasserdampf und/oder Kreisgas mitverwendet.

Mit den im Stand der Technik üblichen Katalysatoren und Aufarbeitungsverfahren (vgl. EP-A 700714, DE-A 19910508, EP-A 700893, DE-A 19910506, EP-A 982287, EP-A 982289, DE-A 19924532,

DE-A 10115277, EP-A 982288) weist das Kreisgas in typischer Weise folgende Zusammensetzungen auf:

3 5 Vol.-% molekularer Sauerstoff,
1 - 5 Vol.-% Wasserdampf,
0 - 3 Vol.-% Kohlenmonoxid,
0 - 8 Vol.-% Kohlendioxid,
0 - 2 Vol.-% Propan,
0,1 - 0,5 Vol.-% Propylen und
85 - 95 Vol.-% molekularer Stickstoff.

Da das Explosionsverhalten von Propan und Propylen vergleichbar ist und sich Wasserdampf und Stickstoff in kaum unterscheidbarer Weise auf das Explosionsdiagramm von Propan und/oder Propen auswirken, kommen als ein im erfindungsgemäßen Sinn im Rechner zu hinterlegendes charakteristisches Explosionsdiagramm z.B. in Betracht:

a) das Diagramm Propylen / $O_2$ - $N_2$;
b) das Diagramm Propan / $O_2$ - $N_2$;
c) das Diagramm Propylen / $O_2$ - $H_2O$;
d) das Diagramm Propan / $O_2$ - $H_2O$;
e) das Diagramm Propylen / $O_2$ - ($N_2/H_2O$);
f) das Diagramm Propan / $O_2$ - ($N_2/H_2O$);

Erfindungsgemäß bevorzugt wird man das Explosionsdiagramm Roh-Propen/$O_2$-$N_2$ im Rechner hinterlegen.

Als Temperatur wird man bei der experimentellen Bestimmung des Explosionsdiagramms eine Temperatur wählen, die nicht zu weit von dem Temperaturbereich entfernt liegt, der bei der Partialoxidation überstrichen wird. Je nach verwendetem Katalysator können diese Reaktionstemperaturen 300°C bis 400°C betragen (eine Temperaturerhöhung um 100°C verschiebt die Explosionsgrenze um etwa 10 % nach unten). Bevorzugt wird man die Temperatur wählen, die das Beschickungsgasgemisch bei Eintritt in die Katalysatorbeschickung aufweist. Der Eingangsdruck liegt bei der in Rede stehenden Partialoxidation normalerweise bei 1 bis 5 bar. Da der Einfluss des Druckes auf den Verlauf der Explosionsgrenze ein vergleichsweise weniger ausgeprägter ist, kann das erfindungsgemäß zu hinterlegende Explosionsdiagramm der Einfachheit halber bei 1 bar ermittelt werden. Bevorzugt wird man jedoch den Eingangsdruck in den Oxidationsreaktor wählen.

Luft, Roh-Propen sowie Kreisgas und/oder Wasserdampf werden dem relevanten Oxidationsreaktor üblicherweise so zugeführt, dass man sie aus getrennten Leitungen kommend zunächst in einem, z.B. statischen (Raum mit Einbauten die Turbulenzen erzeugen), Mischer führt, in welchem sie homogen vermischt und nachfolgend, gegebenenfalls auf Eingangstemperatur erwärmt, dem Oxidationsreaktor zugeführt werden (der Eintritt der Einzelgase in die dem statischen Mischer zugeführte Leitung wird dabei in zweckmäßiger Weise in der Abfolge Kreisgas und/oder Dampf, Roh-Propen, Luft durchgeführt).

Zur Errechnung eines aussagekräftigen aktuellen Betriebspunktes des Beschickungsgasgemisches im Explosionsdiagramm ist z.B. die experimentelle Bestimmung der nachfolgenden Messgrößen ausreichend:

a) die dem Mischer pro Zeiteinheit zugeführte Menge an Luft in $Nm^3$;

b) die dem Mischer pro Zeiteinheit zugeführte Menge an Roh-Propen in $Nm^3$;

c) die dem Mischer pro Zeiteinheit zugeführte Menge an Wasserdampf und/oder Kreisgas in $Nm^3$;

d) der $O_2$-Gehalt des Kreisgas.

Der Sauerstoff- und Stickstoffgehalt der Luft ist bekannt, die Roh-Propen-Menge und die gegebenenfalls mitverwendete Wasserdampf-Menge ergeben sich als unmittelbares Messergebnis und das Kreisgas wird, abgesehen von seinem Sauerstoffgehalt, ausschließlich aus Stickstoff bestehend angenommen. Sollte das Kreisgas noch brennbare Bestandteile enthalten, wirken sich dies für die Frage der Sicherheit nicht nachteilig aus, da ihr Beisein im Explosionsdiagramm lediglich eine Rechtsverschiebung des realen Betriebspunktes bezüglich des errechneten Betriebspunktes bedeuten würde. Im Kreisgas in geringen Mengen enthaltener Wasserdampf bzw. enthaltene Kohlenoxide können in Bezug auf Sicherheitsrelevanz als Stickstoff gewertet werden.

Die Mengenmessung der dem Reaktor zugeführten Gasströme kann mit jedem dazu geeigneten Messgerät vorgenommen werden. Als solche Messgeräte kommen z.B. alle Durchflussmessgeräte wie Drosselgeräte (z.B. Blenden oder Venturirohre), Verdrängungsdurchflussmesser, Schwebekörper-, Turbinen-, Ultraschall-, Wirbel- und Massendurchflussgeräte in Betracht. Aus Gründen des geringen Druckverlustes sind Venturirohre erfindungsgemäß bevorzugt. Durch Berücksichtigung von Druck und Temperatur können die gemessenen Volumenströme in $Nm^3$ umgerechnet werden.

Die Bestimmung des Sauerstoffgehaltes im Kreisgas kann z.B. inline wie in der DE-A 10117678 beschrieben vorgenommen werden. Sie kann prinzipiell aber auch on-line durchgeführt werden, indem man dem aus der Partialoxidation kommenden Produktgasgemisch vorab seines Eintritts in die Zielproduktabtrennung (Aufarbeitung) eine Probe entnimmt und on-line so analysiert, dass die Analyse in einem Zeitraum erfolgt, der kürzer ist als die Verweilzeit des Produktgasgemisches in der Aufarbeitung. D.h., die Gasmenge zum Analysengerät muss über einen Analysengasbypass entsprechend groß und das Rohrleitungssystem zum Analysengerät entsprechend klein gewählt werden. Selbstredend könnte auch eine $O_2$-Bestimmung im Beschickungsgas anstelle der Kreisgasanalyse vorgenommen werden. Natürlich kann auch beides durchgeführt werden. In anwendungstechnisch zweckmäßiger Weise ist die Ermittlung des Betriebspunktes zur Anwendung der erfindungsgemäßen sicherheitsgerichteten speicherprogrammierten Steuerung (SSPS) wenigstens dreikanalig aufgebaut.

D.h., jede Mengenmessung wird mittels drei hintereinander oder parallel geschalteter Fluid Flow Indicator (FFI) durchgeführt. Das gleiche trifft auf die $O_2$-Analyse zu. Unterschreitet einer der drei aus den drei Datensätzen errechneten Betriebspunkte des Beschickungsgasgemisches im Explosionsdiagramm den vorgegebenen Mindestabstand, wird der Gaszufluss z.B. in der Reihenfolge Luft, mit Zeitverzögerung Propen und abschließend, gegebenenfalls Wasserdampf und/oder Kreisgas automatisch geschlossen.

Unter dem Aspekt einer späteren Wiederinbetriebnahme kann es zweckmäßig sein, Wasserdampf und/oder Kreisgas im Kreis weiterzufahren.

Alternativ kann aus den drei einzelnen Messungen auch ein mittlerer Betriebspunkt im Explosionsdiagramm errechnet werden. Unterschreitet dessen Abstand zur Explosionsgrenze einen Mindestwert, erfolgt wie vorstehend beschrieben eine automatische Abschaltung.

Ein typischer stationärer Betriebspunkt des erfindungsgemäßen Verfahrens kann z.B. wie folgt aussehen (alle Mengen in $Nm^3$):

Luft : polymer gerade Propylen : Kreisgas : Wasserdampf = 6,9 : 1 : 8,5 : 0,

wobei das Kreisgas wie folgt zusammengesetzt ist:

| Komponente | Vol.-% |
|---|---|
| Sauerstoff | 3,3 |
| Wasser | 1,5 |
| Kohlenmonoxid | 0,8 |
| Kohlendioxid | 1,6 |
| Propan | 0,3 |
| Propen | 0,3 |
| Acrolein | 0,05 |
| Stickstoff | 92,15, |

oder = 64,8 : 7,6 : 2,5 : 8,5,

oder = 64 : 6 : 0 : 34.

Prinzipiell kann das erfindungsgemäße Verfahren nicht nur für den stationären Betrieb, sondern auch für das An- und Abfahren der Partialoxidation angewendet werden.

Für die Errechnung der Beschickungsgasgemischbetriebspunkte aus den Messwerten für die einzelnen Mengenströme bedarf es jedoch sehr genauer und verlässlicher Messungen.

Da beim Anfahren einer heterogen katalysierten Gasphasenpartialoxidation jedoch anwendungstechnisch bedingt mit, verglichen mit den Mengen, für die die Produktionsanlage ausgelegt ist, geringen Gasmengen gestartet wird und die Messwerte von Mengenmessgeräten insbesondere im ersten Drittel ihres Messbereichs vergleichsweise unzuverlässig sind, wird beim Starten der Partialoxidation in der Regel so verfahren:

Die Freigabe zum Öffnen der Zufuhr von zunächst Luft und dann Propen wird erst erteilt, wenn die Zuflussmengeides Verdünnungsgases (Wasserdampf und/oder Kreisgas) auf einen Mindestwert angestiegen ist, der z.B. 70 % der maximal möglichen Luftzufuhrmenge beträgt. Auf diese Weise können Propen und Luft in beliebigen Verhältnissen zugemischt werden, ohne dass die Sauerstoffgrenzkonzentration überschritten wird. Erst wenn die Luftzuflussmenge das erste Drittel ihres vorgesehenen Maximalwertes überschreitet, wird durch die sicherheitsgerechte speicherprogrammierte Steuerung (SSPS) die Betriebspunktüberwachung aktiviert. Die EP-B 1180508 beschreibt zwar eine alternative Methode zum Anfahren eines Reaktors mit erhöhter Sicherheit. Diese setzt allerdings eine genaue Erfassung der relevanten Gasströme im unteren Messbereich der Messgeräte voraus, wie sie durch die physikalischen Grundprinzipien, die den Mengenmessungen zugrunde liegen, in der Regel nicht gegeben ist. Das Abfahren der heterogen katalysierten Gasphasenpartialoxidation ist demgegenüber weniger kritisch, wenn als erstes die Luftzufuhr geschlossen wird.

In völliger Entsprechung zur vorstehend ausgeführten heterogen katalysierten Partialoxidation von Propylen zu Acrolein und/oder Acrylsäure kann auch bei anderen heterogen katalysierten Partialoxidationen verfahren werden, wie z.B. die heterogen katalysierte Partialoxidation von iso-Buten zu Methacrolein und/oder Methacrylsäure (einstufig, oder die erste Stufe eines zweistufigen Verfahrens), eine einstufige heterogen katalysierte Partialoxidation von Propan zu Acrolein und/oder Acrylsäure, eine einstufige heterogen katalysierte Partialoxidation von iso-Butan zu Methacrolein und/oder Methacrylsäure, eine einstufige heterogen katalysierte Partialoxidation von Alkylen zu Alkylenoxid (z.B. Ethylenoxid oder Propylenoxid), eine einstufige heterogen katalysierte Partialoxidation von Acrolein zu Acrylsäure etc.. Dies gilt insbesondere dann, wenn das insgesamt mitverwendete inerte Verdünnungsgas zu $\geq$ 90 Vol.-% bzw. zu $\geq$ 95 Vol.-% aus nichtbrennbaren inerten Verdünnungsgasen besteht.

Es ist aber auch dann in hervorragender Weise anwendbar, wenn wie bei den in der DE-A 10131297, der DE-A 10219685, der DE-A 10219686 und der WO 01/96270 beschriebenen Verfahren die partiell zu oxidierende Verbindung ein Alkylen ist und als inertes Verdünnungsgas neben nicht brennbaren Gasen auch ein brennbares inertes Verdünnungsgas, nämlich das zum Alkylen homologe Alkan (z.B. bei Propylen das Propan, bei iso-Buten das iso-Butan) mitverwendet wird.

Da die Explosionsdiagramme Alkylen/$O_2$ - $N_2$ bzw. homologes Alkan/$O_2$ - $N_2$ kaum voneinander verschieden sind, kann eines von beiden als charakteristisches Explosionsdiagramm im Rechner hinterlegt werden. Zusätzlich vereinfacht wird das erfindungsgemäße Verfahren dabei dann, wenn das Kreisgas in die der Partialoxidation vorgeschaltete katalytische Dehydrierung so rückgeführt wird, so daß bei der Partialoxidation $N_2$ und das homologe Alkan im wesentlichen die einzigen inerten Verdünnungsgase sind.

Das erfindungsgemäße Verfahren ist aber auch dann anwendbar, wenn als inertes Verdünnungsgas nur brennbare inerte Verdünnungsgase eingesetzt werden.

Gegebenenfalls ist es dazu erforderlich, die Anzahl der Komponentenachsen im Explosionsdiagramm auf mehr als zwei zu erweitern, wie es die Figur in der DE-A 19902562 vom Prinzip her ausweist. Im Fall von Ammoxidationen wird der Ammoniak ins Explosionsdiagramm mit einbezogen.

Das erfindungsgemäße Verfahren läßt sich auf die zweite Stufe einer zweistufigen heterogen katalysierten Gasphasenoxidation im Rohrbündelreaktor von Propen zu Acrylsäure oder von iso-Buten zu Methacrylsäure auch dann anwenden, wenn diese zweite Stufe als Beschickungsgasgemisch unmittelbar das Produktgasgemisch der ersten Stufe und diesem (in einem Mischer) zugesetzte Luft (Sekundarluft) enthält. Der Betriebspunkt des Beschickungsgasgemisches der zweiten Stufe läßt sich dann in einfacher Weise aus dem wie beschrieben ermittelbaren Betriebspunkt des Beschickungsgasgemisches für die erste Stufe und dessen Menge sowie der Sekundärluftmenge ermitteln. Dies deshalb, weil die Katalysatoreigenschaften unter den für die erste Stufe gewählten Reaktionsbedingungen bekannt sind (Propylenumsatz normalerweise $\geq$ 90 bzw. $\geq$ 95 mol-%, Selektivität der Acroleinbildung $\geq$ 90 mol-%, verbleibende Nebenproduktselektivität im wesentlichen Kohlenoxid- und Wasserdampfbildung), so daß aus dem Beschickungsgasgemisch für die erste Stufe ohne zusätzliche Meßnotwendigkeit die Zusammensetzung des die erste Stufe verlassenden Produktgasgemisches rechnerisch ermittelt werden kann.

Im einfachsten Fall wird dabei als charakteristisches Explosionsdiagramm das Diagramm Acrolein/$O_2$ - $N_2$ hinterlegt.

Ein Beisein von CO bewirkt in selbigem eine Verschiebung des Verlaufs der Explosionsgrenze nach rechts. Bezüglich Druck und Temperatur kann analog zur ersten Stufe verfahren werden. Die Reaktionstemperaturen liegen hier je nach verwendetem Katalysator bei 230 bis 300°C. Der Eintrittsdruck beträgt auch in der zweiten Stufe in der Regel 1 bis 5 bar.

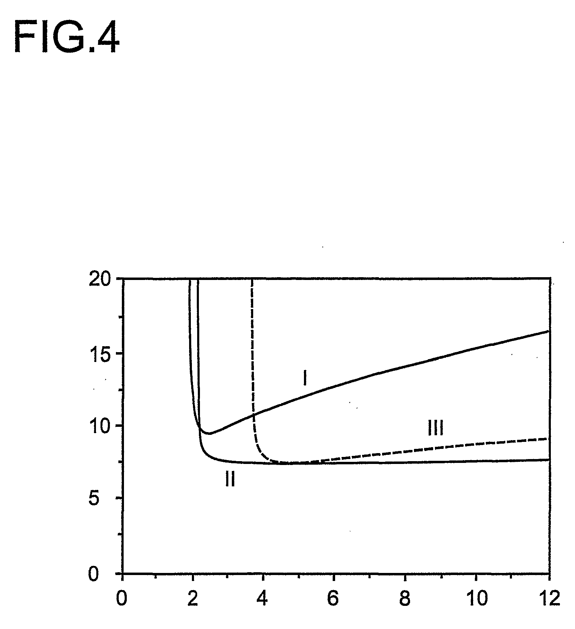

Figur 4 zeigt repräsentative Explosionsdiagramme für die Systeme Propylen/$O_2$ - $N_2$ (I), Acrolein/$O_2$ - $N_2$ (II) und Acrolein, CO/$O_2$ - $N_2$ (III) (dabei wurde die CO Konzentration konstant bei 1,6 Vol:-% gehalten).

Alternativ zum erfindungsgemäßen Verfahren wurde auch schon vorgeschlagen, für die zweite Stufe neben dem Kontrollsystem für die erste Stufe als Abschaltgröße lediglich eine maximal Menge an Sekundärluft, oder ein maximales Verhältnis der Mengen von Sekundarluft/Primärluft (der ersten Stufe zugeführte Luft), oder eine maximale Sauerstoffkonzentration im Produktgasgemisch der zweiten Stufe zu verwenden. Insbesondere der letzte Abschaltmechanismus vermag jedoch nicht zu befriedigen, da er erst zeitverzögert reagiert, nämlich dann, wenn die Fehlzufuhr die zweite Stufe verlässt. Der Abschaltmechanismus wirkt hier in erster Linie auf die Sekundärluftzufuhr.

Abschließend sei festgehalten, daß ein Abschaltszenario für die zweite Stufe auch auf der Grundlage der beiden folgenden Abschaltgrößen denkbar ist: maximales Verhältnis von der zweiten Stufe zugeführter Sekundärluftmenge zu der ersten Stufe zugeführter Roh-Propylenmenge und maximale Propylenkonzentration im Produktgasgemisch der zweiten Stufe.

Schließlich sei noch erwähnt, daß es mit dem erfindungsgemäßen Verfahren problemlos möglich ist, heterogen katalysierte Propylenpartialoxidationen zu Acrolein und/oder Acrylsäure durchzuführen, deren Beschickungsgasgemisch bis zu 10 Vol.-% und mehr an Propylen, bei einem molaren Verhältnis von $O_2$/Propylen > 1, enthält.

Die Belastung der Katalysatoren mit Beschickungsgasgemisch kann wie in der DE-A 19910508 oder der DE-A 19910506 gewählt werden. Die Aufarbeitung zur Kreisgasgewinnung kann wie in der WO 97/48669 erfolgen.

Kontinuierlicher Betrieb bedeutet in dieser Schrift, daß dem Reaktor über einen längeren Zeitraum, in der Regel wenigstens einige Tage, ohne Unterbrechung Ausgangsgasgemisch kontinuierlich zugeführt und Produktgasgemisch kontinuierlich entnommen wird.

**Patentansprüche**

1. Verfahren zum sicheren Betreiben einer kontinuierlich betriebenen heterogen katalysierten Gasphasen-Partialoxidation wenigstens einer organischen Verbindung in einem Oxidationsreaktor, dessen Beschickungsgasgemisch neben der wenigstens einen partiell zu oxidierenden organischen Verbindung und molekularem Sauerstoff als Oxidationsmittel wenigstens ein sich unter den Bedingungen der heterogen katalysierten Gasphasen-Partialoxidation im wesentlichen inert verhaltendes Verdünnungsgas umfaßt, bei dem mittels eines Abschaltmechanismus verhindert wird, daß der Oxidationsreaktor mit einem Beschickungsgasgemisch beschickt wird, dessen Zusammensetzung eine explosive ist, **dadurch gekennzeichnet, daß** der Abschaltmechanismus wie folgt gestaltet ist:

a) in einem Rechner wird ein für das Beschickungsgasgemisch charakteristisches Explosionsdiagramm hinterlegt, in welchem in Abhängigkeit von der Zusammensetzung des Beschickungsgasgemisches explosive und nicht explosive Zusammensetzungen gegeneinander abgegrenzt sind;

b) durch Bestimmungen bezüglich Menge und gegebenenfalls Zusammensetzung der dem Reaktor zur Erzeugung des Beschickungsgasgemisches zugeführten Gasströme wird ein Datensatz ermittelt, der an den Rechner weitergeleitet wird;

c) aus dem unter b) erhaltenen Datensatz errechnet der Rechner einen aktuellen Betriebspunkt des Beschickungsgasgemisches im Explosionsdiagramm;

d) fällt der Abstand des Betriebspunktes zur nächstliegenden Explosionsgrenze unter einen vorgegebenen Mindestwert, wird die Zufuhr von Gasströmen zum Reaktor automatisch unterbrochen,

wobei der Sauerstoffgehalt des Beschickungsgasgemisches oberhalb der Sauerstoffgrenzkonzentration des Beschickungsgasgemisches liegt.

2. Verfahren nach Anspruch 1, **dadurch gekennzeichnet, daß** die wenigstens eine organische Verbindung Propylen, iso-Buten, Ethylen, Propan, iso-Butan, Acrolein oder Methacrolein ist.

3. Verfahren nach Anspruch 1, **dadurch gekennzeichnet, daß** der Sauerstoffgehalt des Beschickungsgasgemisches $\geq$ 0,5 Vol.-%-Punkte oberhalb der Sauerstoffgrenzkonzentration des Beschickungsgasgemisches liegt.

4. Verfahren nach einem der Ansprüche 1 bis 3, **dadurch gekennzeichnet, daß** als Sauerstoffquelle Luft verwendet wird.

5. Verfahren nach einem der Ansprüche 1 bis 4, **dadurch gekennzeichnet, daß** das inerte Verdünnungsgas zu $\geq$ 90 Vol.-% aus nicht brennbaren Bestandteilen besteht.

6. Verfahren nach einem der Ansprüche 1 bis 5, **dadurch gekennzeichnet, daß** das inerte Verdünnungsgas zu $\geq$ 95 Vol.-% aus nicht brennbaren Bestandteilen besteht.

7. Verfahren nach einem der Ansprüche 1 bis 5, **dadurch gekennzeichnet, daß** das inerte Verdünnungsgas zu $\geq$ 90 Vol.-% aus $N_2$, $H_2O$ und/oder $CO_2$ besteht.

8. Verfahren nach einem der Ansprüche 1 bis 5, **dadurch gekennzeichnet, daß** das inerte Verdünnungsgas zu $\geq$ 95 Vol.-% aus $N_2$, $H_2O$ und/oder $CO_2$ besteht.

9. Verfahren nach einem der Ansprüche 1 bis 8, **dadurch gekennzeichnet, daß** als eine Quelle des inerten Verdünnungsgases Kreisgas verwendet wird.

10. Verfahren nach einem der Ansprüche 1 bis 9, **dadurch gekennzeichnet, daß** zur Bestimmung der Mengen der dem Reaktor zugeführten Gasströme Venturirohre verwendet werden.

11. Verfahren nach einem der Ansprüche 1 bis 10, **dadurch gekennzeichnet, daß** als Sauerstoffquelle Luft verwendet wird und dann, wenn der Abstand des Betriebspunktes zur nächstliegenden Explosionsgrenze unter den vorgegebenen Mindestwert fällt, als erster Gasstrom die Luftzufuhr zum Reaktor unterbrochen wird.

12. Verfahren nach einem der Ansprüche 1 bis 11, **dadurch gekennzeichnet, daß** die wenigstens eine organische Verbindung Propylen und das charakteristische Explosionsdiagramm das Explosionsdiagramm des Gemisches Propylen/$O_2$-$N_2$ ist.

13. Verfahren nach einem der Ansprüche 1 bis 12, **dadurch gekennzeichnet, daß** der Mindestwert aus einer statistischen Fehlerbetrachtung der zur Berechnung des Betriebspunktes notwendigen Meßgrößen berechnet wird.

14. Verfahren nach einem der Ansprüche 1 bis 13, **dadurch gekennzeichnet, daß** der Rechner eine sicherheitsgerichtete speicherprogrammierte Steuerung ist (SSPS).

**Claims**

1. A process for the safe operation of a continuously operated heterogeneously catalyzed gas-phase partial oxidation of at least one organic compound in an oxidation reactor whose feed gas mixture comprises, in addition to the at least one organic compound to be partially oxidized and molecular oxygen as an oxidizing agent, at least one diluent gas substantially inert under the conditions of the heterogeneously catalyzed gas-phase partial oxidation, in which a cut-out mechanism is used to prevent the oxidation reactor from being fed with a feed gas mixture whose composition is an explosive one, wherein the cut-out mechanism is designed as follows:

a) an explosion diagram which is characteristic of the feed gas mixture and in which a distinction is made between explosive and nonexplosive compositions as a function of the composition of the feed gas mixture is deposited in a computer;
b) a data record is established by determination with respect to the amount and, if required, composition of the gas streams fed to the reactor for producing the feed gas mixture, and is transmitted to the computer;
c) the computer calculates an actual operating point of the feed gas mixture in the explosion diagram from the data record obtained under b);
d) if the distance from the operating point to the nearest explosion limit is below a predetermined minimum value, the feed of gas streams to the reactor is automatically stopped,

wherein the oxygen content of the feed gas mixture is above the limiting oxygen concentration of the feed gas mixture.

2. The process according to claim 1, wherein the at least one organic compound is propylene, isobutene, ethylene, propane, isobutane, acrolein or methacrolein.

3. The process according to claim 1, wherein the oxygen content of the feed gas mixture is $\geq$ 0.5% by volume above the limiting oxygen concentration of the feed gas mixture.

4. The process according to any of claims 1 to 3, wherein the oxygen source used is air.

5. The process according to any of claims 1 to 4, wherein the inert diluent gas comprises $\geq$ 90% by volume of nonflammable components.

6. The process according to any of claims 1 to 5, wherein the inert diluent gas comprises $\geq$ 95% by volume of nonflammable components.

7. The process according to any of claims 1 to 5, wherein the inert diluent gas comprises $\geq$ 90% by volume of $N_2$, $H_2O$ and/or $CO_2$.

8. The process according to any of claims 1 to 5, wherein the inert diluent gas comprises $\geq$ 95% by volume of $N_2$, $H_2O$ and/or $CO_2$.

9. The process according to any of claims 1 to 8, wherein recycle gas is used as a source of the inert diluent gas.

10. The process according to any of claims 1 to 9, wherein Venturi tubes are used for determining the amounts of the gas streams fed to the reactor.

11. The process according to any of claims 1 to 10, wherein the oxygen source used is air and the air feed to the reactor is stopped when the distance from the operating point to the nearest explosion limit is below the predetermined minimum value.

12. The process according to any of claims 1 to 11, wherein the at least one organic compound is propylene and the characteristic explosion diagram is the explosion diagram of the mixture propylene/$O_2$-$N_2$.

13. The process according to any of claims 1 to 12, wherein the minimum value is calculated from a statistical error evaluation of the measured quantities required for calculating the operating point.

14. The process according to any of claims 1 to 13, wherein the computer is a safety programmable logic controller (SPLC).

**Revendications**

1. Procédé pour faire fonctionner de façon sûre une oxydation partielle en phase gazeuse à catalyse hétérogène, fonctionnant en continu, d'au moins un composé organique dans un réacteur d'oxydation dont le mélange de gaz d'alimentation comprend, outre ledit au moins un composé organique à oxyder partiellement et de l'oxygène moléculaire en tant qu'oxydant, au moins un gaz de dilution se comportant de façon essentiellement inerte dans les conditions de l'oxydation partielle en phase gazeuse à catalyse hétérogène, dans lequel on empêche au moyen d'un mécanisme d'arrêt l'alimentation du réacteur d'oxydation avec un mélange de gaz d'alimentation dont la composition est une composition explosive, **caractérisé en ce que** le mécanisme d'arrêt est constitué comme suit :

   a) on met en mémoire dans une unité de calcul un diagramme d'explosion, caractéristique du mélange de gaz d'alimentation, dans lequel des compositions explosives et des compositions non explosives sont délimitées les unes par rapport aux autres en fonction de la composition du mélange de gaz d'alimentation ;
   b) par des déterminations concernant la quantité et éventuellement la composition des flux de gaz envoyés au réacteur pour la production du mélange de gaz d'alimentation, est généré un ensemble de données qui est transmis à l'unité de calcul ;
   c) l'unité de calcul calcule détermine par le calcul à partir de l'ensemble de données obtenu en b) un point de fonctionnement actuel du mélange de gaz d'alimentation dans le diagramme d'explosion ;
   d) si l'espacement entre le point de fonctionnement et la limite d'explosion la plus voisine est inférieur à une valeur minimale préétablie, l'envoi des flux de gaz au réacteur est automatiquement interrompu,

   la teneur en oxygène du mélange de gaz d'alimentation étant supérieure à la concentration limite d'oxygène du mélange de gaz d'alimentation.

2. Procédé selon la revendication 1, **caractérisé en ce que** ledit au moins un composé organique est le propylène, l'isobutène, l'éthylène, le propane, l'isobutane, l'acroléine ou la méthacroléine.

3. Procédé selon la revendication 1, **caractérisé en ce que** la teneur en oxygène du mélange de gaz d'alimentation se situe à $\geq$ 0,5 point en % en volume au-dessus de la concentration limite d'oxygène du mélange de gaz d'alimentation.

4. Procédé selon l'une quelconque des revendications 1 à 3, **caractérisé en ce qu'**en tant que source d'oxygène on utilise de l'air.

5. Procédé selon l'une quelconque des revendications 1 à 4, **caractérisé en ce que** le gaz de dilution inerte consiste à raison de $\geq$ 90 % en volume en constituants non combustibles.

6. Procédé selon l'une quelconque des revendications 1 à 5, **caractérisé en ce que** le gaz de dilution inerte consiste à raison de $\geq$ 95 % en volume en constituants non combustibles.

7. Procédé selon l'une quelconque des revendications 1 à 5, **caractérisé en ce que** le gaz de dilution inerte consiste à raison de $\geq$ 90 % en volume en $N_2$, $H_2O$ et/ou $CO_2$.

8. Procédé selon l'une quelconque des revendications 1 à 5, **caractérisé en ce que** le gaz de dilution inerte consiste à raison de $\geq$ 95 % en volume en $N_2$, $H_2O$ et/ou $CO_2$.

9. Procédé selon l'une quelconque des revendications 1 à 8, **caractérisé en ce qu'**en tant que source du gaz de dilution inerte on utilise du gaz en circuit.

10. Procédé selon l'une quelconque des revendications 1 à 9, **caractérisé en ce que** pour la détermination des quantités des flux de gaz envoyés au réacteur on utilise des tubes de Venturi.

11. Procédé selon l'une quelconque des revendications 1 à 10, **caractérisé en ce qu'**en tant que source d'oxygène on utilise de l'air et ensuite, lorsque l'espacement entre le point de fonctionnement et la limite d'explosion la plus voisine devient inférieur à la valeur minimale préétablie, l'envoi d'air au réacteur, en tant que premier flux de gaz, est interrompu.

12. Procédé selon l'une quelconque des revendications 1 à 11, **caractérisé en ce que** ledit au moins un composé organique est le propylène et le diagramme d'explosion caractéristique est le diagramme d'explosion du mélange propylène/$O_2$-$N_2$.

13. Procédé selon l'une quelconque des revendications 1 à 12, **caractérisé en ce que** la valeur minimale est calculée à partie d'une analyse statistique d'erreurs des grandeurs mesurées requises pour le calcul du point de fonctionnement.

14. Procédé selon l'une quelconque des revendications 1 à 13, **caractérisé en ce que** l'unité de calcul est un automate programmable de sécurité (APS).

# FIG.1

# FIG.2

# FIG.3

# FIG.4

EP 1 525 172 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 731080 A
- DE 19622331 A
- DE 2351151 A
- DE 2526238 A
- EP 92097 A
- EP 58927 A
- DE 4132263 A
- DE 4132684 A
- DE 4022212 A
- EP 522871 A
- DE 2106796 A
- DE 1624921 A
- GB 1464198 A
- GB 1291354 A
- DE 2025430 A
- DE AS1254137 B
- DE 2159346 A
- EP 372972 A
- WO 890710 A
- DE 4311608 A
- DE 10131297 A
- EP 1090684 A

- EP 608838 A
- DE 10046672 A
- EP 529853 A
- WO 0196270 A
- DE 10028582 A
- EP 1180508 A
- DE 19902562 A
- EP 1180508 B
- EP 700893 A
- EP 700714 A
- DE 19910508 A
- DE 19919596 A
- DE 19910506 A
- EP 982287 A
- EP 982289 A
- DE 19924532 A
- DE 10115277 A
- EP 982288 A
- DE 10117678 A
- DE 10219685 A
- DE 10219686 A
- WO 9748669 A

**In der Beschreibung aufgeführte Nicht-Patentliteratur**

- **BEYER.** Lehrbuch der organischen Chemie. Hirzel Verlag Stuttgart, 1973, 261

20